# EUROPEAN PATENT SPECIFICATION

(11) **EP 4 433 113 B1**
(45) Date of publication and mention of the grant of the patent: **14.05.2025**
(21) Application number: 22817181.5
(22) Date of filing: 10.11.2022
(51) Int. Cl.: A61M 1/06

(54) **SENSOR ARRANGEMENT FOR A BREAST PUMP DEVICE AND BREAST PUMP DEVICE USING THE SENSOR ARRANGEMENT**
SENSORANORDNUNG FÜR EINE BRUSTPUMPENVORRICHTUNG UND BRUSTPUMPENVORRICHTUNG MIT VERWENDUNG DER SENSORANORDNUNG
AGENCEMENT DE CAPTEUR POUR UN DISPOSITIF DE TIRE-LAIT ET DISPOSITIF DE TIRE-LAIT UTILISANT L'AGENCEMENT DE CAPTEUR

(30) Priority: 18.11.2021 EP 21208923
(43) Date of publication of application: 25.09.2024
(73) Proprietor: Koninklijke Philips N.V., 5656 AG Eindhoven (NL)
(72) Inventor: JOOSTEN, Franciscus Ivo, 5656 AG Eindhoven (NL)
(74) Representative: Philips Intellectual Property & Standards
(86) International application number: PCT/EP2022/081375
(87) International publication number: WO 2023/088758

(56) References cited:
- US-A1- 2019 328 945
- US-A1- 2020 016 306
- US-A1- 2021 268 158

## Description

### FIELD OF THE INVENTION

The invention relates to a breast pump device and a method of operating a breast pump device, and in particular relates to a sensor arrangement for use as part of the breast pump device.

### BACKGROUND OF THE INVENTION

Breast pumps are used by breast feeding women to extract milk from their breast such that the extracted milk can be fed to their babies at a later time.

It is well known that best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

However, at the moment, the transition from the stimulation mode to the expression mode is not based on detection of MER, it is either time-based, i.e. a fixed time after switching on, or manual by pushing a button. Both these methods have drawbacks. The timing method is based on an average time, so late for a lot of women and too early for others. The manual method has the drawback that not every woman feels that the milk is flowing and they have to check the bottle. Therefore, detection when milk starts flowing into the bottle would allow for a personalized solution for automatic switching between the stimulation mode and the expression mode.

It would be desirable to be able to detect the first drop or drops, to enable automatic switching between a stimulation mode and an expression mode.

WO 2019/149486 discloses a breast pump which includes a sensor arrangement for detecting a volume of expressed milk. The sensor arrangement is used to measure a free fall time of milk droplets, and thereby determine a liquid level of the milk collection vessel into which the milk droplets are falling. It is also recognized that the start of the milk let-down reflex can be detected based on a sudden increase in volume of collected milk. The pump settings of the breast pump may then be adjusted.

However, this approach requires a volume of milk to be collected before detection is possible of a change in the rate at which the volume increases. Thus, it is not suitable for detecting the first drop or drops of expressed milk.

US 201/328945 discloses a sensor for measuring a liquid level in a milk collection vessel of a breast pump, based on a time of flight sensor.

US 2021/268158 discloses a breast pump which includes a level sensor. The level sensor is again based on time of flight for a signal reflected off the milk surface. Multiple sensors enable the milk volume to be determined when the milk container is not horizontal.

US 2020/0016306 discloses a breast shield and a time of flight sensor for measuring milk volume, based on the distance to the milk level.

There is therefore a need for an improved sensor arrangement for early detection of the initial milk expression.

### SUMMARY OF THE INVENTION

The invention is defined by the claims.

According to examples in accordance with the invention, there is provided a collection vessel system for use with a breast pump, comprising:
a collection vessel for collecting milk expressed during use of the breast pump, the collection vessel having a base; and
a sensor system for sensing the start of milk expression into the collection vessel,
wherein the sensor system comprises an emitter for emitting electromagnetic radiation towards the base and a sensor for receiving electromagnetic radiation reflected from the base,
wherein:
   the base of the collection vessel when empty has a first reflectance to the emitted electromagnetic radiation below 20%; or
   the collection vessel system comprises a support on which the collection vessel is mounted in use, and the combination of the base (44) of the collection vessel and the support, when the collection vessel is empty, has a first reflectance to the emitted electromagnetic radiation below 20%, and
   wherein the sensor system is adapted to detecting a change from the first reflectance to a milk reflectance, thereby to identify when there is a change from an empty collection vessel to a collection vessel containing milk.

The invention is thus based on the use of a sensor which can detect the first few drops of milk by detecting a change of reflectance against the bottom of the collection vessel. For example, the base of the collection vessel may have a dark surface appearance when empty (for the example of visible light). More generally, it has a low reflectance (below 10%) to the electromagnetic radiation used by the sensor system. This will be termed "dark" for simplicity. This dark surface reflects only a few percent of the electromagnetic radiation to which it is exposed. When white milk covers the dark bottom of collection vessel, the reflectance increases significantly (to above 20%).

The sensor system is adapted to detect a change from the first reflectance to a milk reflectance. This means that even for a same liquid level, the reflectance change can be identified. Thus, the sensing of the reflectance may be considered to a separate function to a liquid level measurement. Indeed, the sensor system may combine the reflectance detection and time of flight level sensing as explained below.

The emitter is for example for emitting visible light and the sensor is for detecting visible light.

The emitter may instead be for emitting infrared light and the sensor is for detecting infrared light.

The change of reflectance can be measured in several ways, for example by measuring the reflectance directly with visible light or with infrared light. A known amount of light is emitted, and the sensor measures the amount of reflected light.

The sensor system may comprise a time of flight measurement system.

A time of flight sensor can measure a distance based on the time it takes for the electromagnetic radiation to travel to the reflecting surface and bounce back. This can enable a milk level within the collection vessel to be determined as well as the initial presence of milk.

The sensor system is for example adapted to record a time taken to make a time of flight measurement and to derive the change in reflectance from the time taken to make a time of flight measurement.

One option is for the time of flight sensor to measure reflectance directly as well as calculating a time of flight. However, many existing time of flight sensors do not provide access to the raw detection signal. Instead, a time taken to obtain a time of flight measurement may also be used as a proxy for a reflectance measurement. This enables many more off-the-shelf time of flight sensors to be used.

When the reflectance is low, the time of flight sensor needs to make more measurements before a time of flight measurement is generated, hence it takes longer to determine the distance. Thus, rather than directly measuring a light reflectance, a difference in measurement time can be used to indicate the reflectance of the bottom of the container and hence determine if there is milk present or not.

In another example, the sensor system is adapted to derive the change in reflectance from a rate of change of the time taken to make time of flight measurements. Thus, rather than relying on the absolute value of the time taken to make a time of flight measurement, a slope of the change in time taken over time may be used.

The collection vessel for example comprises, or is associated with, an absorbing layer at or beneath the base of the collection vessel. This provides the desired reflectance contrast between an empty collection vessel and one with a small amount of collected milk. If the collection vessel system comprises a support on which the collection vessel is mounted in use, the support for example comprises an absorbing layer.

The sensor system is for example faces the base of the collection vessel. It may be mounted at the top or at a side of the collection vessel.

The sensor system may for example sense the reflectance at a particular location which is preferably the lowest location of the base in use, so that it is where milk will first collect. There may for example be a well for this purpose, so that the sensor system is detected the lowest point of the collection vessel for all intended orientations of the collection vessel in use. Instead, the emitter of the sensor system may provide its output to an area of the base so that milk at any location on the base will result in an increased reflection.

The invention also provides a breast pump device, comprising
at least one breast receiving portion configured to receive a breast of a user; and
the collection vessel system as defined above.

The breast pump device may comprise:
a pressure source coupled to the at least one breast receiving portion; and
a controller configured to control an operation of the pressure source in a stimulation mode and an expression mode,
wherein the controller is configured to switch from a stimulation mode to an expression mode in response to a signal from the sensor system of the collection vessel system.

The invention also provides a method of controlling a breast pump device for the non-therapeutic expression of milk, comprising:
determining the start of milk expression into a collection vessel system when using the breast pump device based on received data from a sensor system which senses a change in reflectance of a base of the collection vessel system to electromagnetic radiation used by the sensor system from below 20% to a reflectance indicative of a collection vessel containing milk; and
generating an output based on the determined start of milk expression.

The method may comprise using the generated output to control the breast pump device to switch from a stimulation mode to an expression mode in response to the determined start of milk expression.

The invention also provides a computer program comprising computer program code which is adapted, when said program is run on the controller of the breast pump device defined above to implement the method defined above.

The invention also provides a controller for determining a start of milk collection into a collection vessel, wherein the controller is adapted to:
receive data from a sensor system, sensing a change in reflectance of a base of the collection vessel to electromagnetic radiation used by the sensor system from below 20% to a reflectance indicative of a collection vessel containing milk; and
interpret the received data to determine the start of milk collection and generate an output based on the determined start of milk collection.

The controller may be adapted to interpret the received data by analyzing convergence times of time of flight sensor data.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the typical parts of a known breast pump system;
Figure 2 shows a wearable breast pump;
Figure 3 shows a first example of a collection vessel system for use with a breast pump;
Figure 4 shows a second example of a collection vessel system for use with a breast pump;
Figure 5 shows a third example of a collection vessel system for use with a breast pump; and
Figure 6 shows a graph of convergence time versus weight of collected milk.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sensor system for sensing the start of milk expression into a collection vessel when using a breast pump device. A sensor is used to provide a signal indicating the presence of the first milk expressed, for use in controlling the breast pump to switch from a stimulation mode to an expression mode. The sensor detects a change in the reflectance of the base of the collection vessel to the electromagnetic sensing radiation used by the sensor.

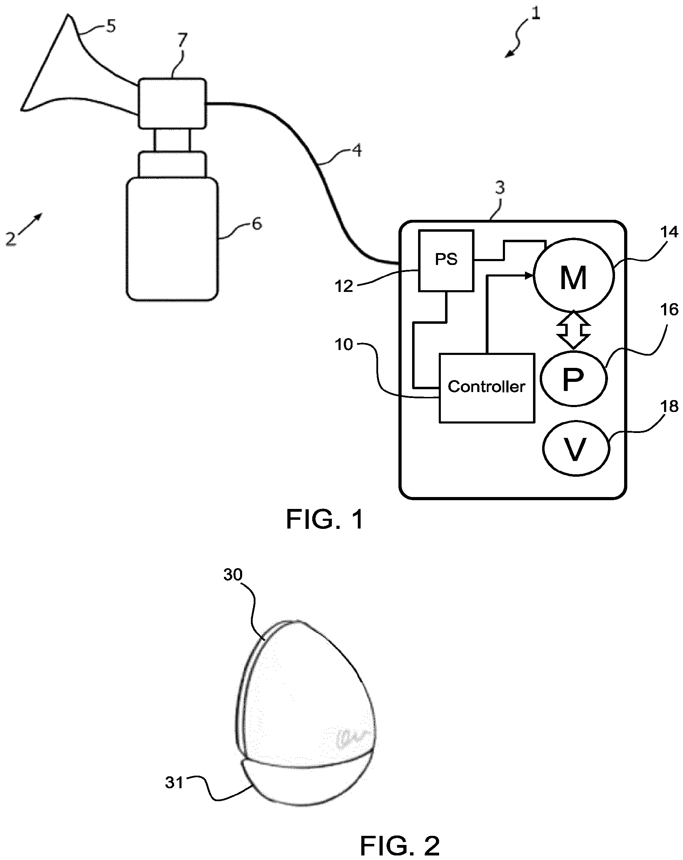

Figure 1 shows a known breast pump system 1, comprising an expression unit 2 and a drive arrangement for controlling the expression function of the breast pump.

In the example shown, the drive arrangement comprises a pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a receptacle 6 for collecting the expressed milk. The funnel 5 and the receptacle 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turns applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection vessel. This enables breast pumping to be performed more discretely.

There are also other types of wearable breast pump whereby the expression unit is fixed to a breast and the pump unit and/or milk container are situated elsewhere on the body of a user.

Figure 2 shows a wearable breast pump, comprising an expression kit 30 attached to a respective milk collection vessel 31.

The invention provides a sensor system for sensing the start of milk expression into the milk collection vessel when using a breast pump device. A sensor is used to provide a signal indicating the presence of the first milk expressed, for use in controlling the breast pump to switch from a stimulation mode to an expression mode.

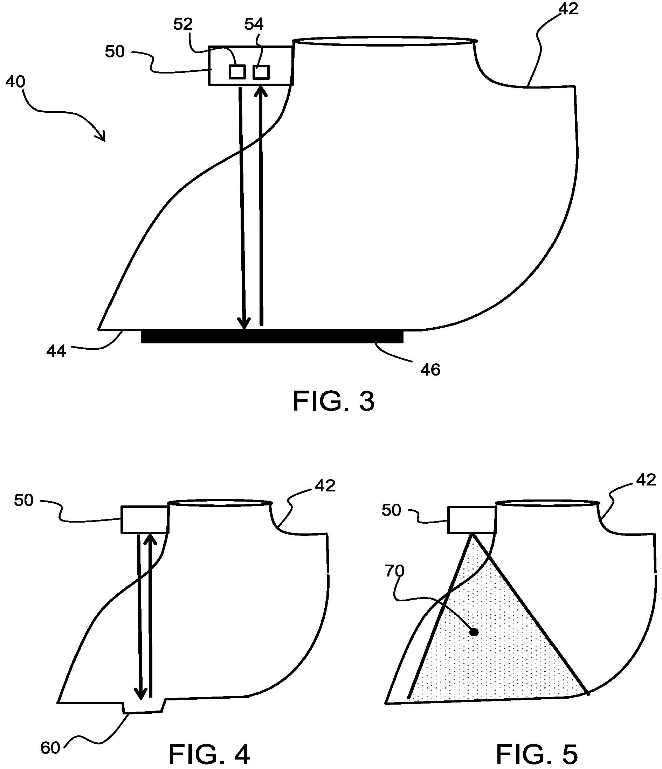

Figure 3 shows a first example of a collection vessel system 40 for use with a breast pump such as shown in Figures 1 or 2.

The collection vessel system 40 comprises a collection vessel 42 for collecting milk expressed during use of the breast pump. The collection vessel 42 has a base 44. In use, the collection vessel may have a single orientation (e.g. it is placed on a horizontal surface) or it may have a range of possible orientations in the case of a wearable system. The base is the lowest surface to which the milk flows in use.

A sensor system 50 is provided for sensing the start of milk expression into the collection vessel 42. The sensor system comprises an emitter 52 for emitting electromagnetic radiation towards the base 44 and a sensor 54 for receiving electromagnetic radiation reflected from the base.

The sensor system is mounted at a top of the collection vessel 42 facing the base 44 of the collection vessel.

The base 44 of the collection vessel 42 when empty has a first, relatively low, reflectance to the emitted electromagnetic radiation, in particular below 10%, whereas the collected milk has a second, relatively high, reflectance to the emitted electromagnetic radiation, in particular above 20%. The sensor system 50 can thereby detect a change in reflectance to identify when there is a change from an empty collection vessel to a collection vessel containing milk.

The sensor system can thus detect the first few drops of milk by detecting a change of reflectance from the bottom of the collection vessel. For example, the base 44 of the collection vessel may have a dark surface appearance when empty, for example provided by an absorbing layer 46 beneath the base, and the vessel itself is transparent to the radiation used by the sensor system.

The absorbing layer may be part of the vessel itself (e.g. an external surface coating) or it may be a layer on which the vessel is seated when the breast pump is assembled. The absorbing layer 46 for example reflects only a small fraction of the electromagnetic radiation to which it is exposed. When white milk covers a part of the dark bottom of the collection vessel 42, the reflectance increases significantly and that increase can be detected by the sensor system.

It has been found that only a thin film of milk is needed to enable detection to take place, for example around 2ml for a typical wearable breast pump container.

The emitter 52 may be a visible light source or an infrared light source, and correspondingly the sensor 54 may be a visible light sensor or an infrared sensor. For example, the sensing may be based on the use of a near infrared LED and suitable detector (which could be a broadband detector with suitable filtering to be selective to the LED frequency).

The change in reflectance can be obtained simply based on a comparison of the intensity of the emitted radiation and intensity of the received reflected radiation.

In order to detect the first drops of expressed milk, the sensor system should be directed to the lowest point of the base of the vessel 42.

Figure 4 shows that a well 60 may be provided as the lowest point, so that the first expressed milk drops flow to a known location, for all intended orientations of the vessel in use.

Figure 5 shows that instead of sensing reflection from a localized point, the emitter and sensor may have a field of view 70 which covers all parts of the base to which the first expressed milk may flow. Milk at any location of the base will result in a reflection. The reflection from the milk surface will be diffuse rather than specular, so a portion of the reflected radiation will be directed back to the sensor, if it has a corresponding field of view.

The sensor system may comprise a time of flight measurement system, which records a time duration for the emitted radiation to return to the sensor. A distance is thus measured based on the time it takes for the electromagnetic radiation to travel to the reflecting surface and back. This can enable a milk level within the collection vessel to be determined as well as the initial presence of milk.

The sensor system may record a time taken to make a time of flight measurement and to derive the change in reflectance from the time taken to make time of flight measurement.

Known time of flight (ToF) sensors perform several measurements in rapid succession and then averages out the data. This all happens in a fully automated manner inside the time of flight sensor.

This time it takes to perform the multiple measurements is for example called the "convergence time" (for example in time of flight sensors of ST microelectronics ^{™}). The convergence time can be looked up in a register of the time of flight sensor.

For example, the time of flight sensor may record a total execution time, which is the sum of a pre-calibration time (e.g. 3.2ms), a range convergence time (variable) and a readout averaging time (e.g. 4.3ms). There is a maximum overall execution time.

It is of interest to use the convergence time because the raw receiver data is not externally accessible in many off-the-shelf time of flight sensors.

By way of example, a time of flight sensor may be the VL6180 proximity sensing module of ST Microelectronics ^{™}. It uses an IR emitter and range sensor and has the pre-calibration and readout averaging times outlined above.

It returns the actual range convergence time (not including the readout averaging time). The range convergence time is dependent on the reflectance and the range of the target, as shown by table 1 below taken from the production data datasheet of the VL6180. The range convergence times are in ms.

**Table 1**

| **Range (mm)** | **Target reflectance** | | | |
|---|---|---|---|---|
| | **3%** | **5%** | **17%** | **88%** |
| 10 | 0.43 | 0.33 | 0.18 | 0.18 |
| 20 | 0.94 | 0.73 | 0.28 | 0.18 |
| 30 | 1.89 | 1.40 | 0.51 | 0.18 |
| 40 | 3.07 | 2.25 | 0.81 | 0.18 |
| 50 | 4.35 | 3.24 | 1.18 | 0.24 |
| 60 | 5.70 | 4.22 | 1.60 | 0.32 |
| 70 | 7.07 | 5.35 | 2.07 | 0.49 |
| 80 | 8.41 | 6.45 | 2.58 | 0.50 |
| 90 | 9.58 | 7.56 | 3.14 | 0.61 |
| 100 | 10.73 | 8.65 | 3.69 | 0.73 |

It can be seen that for a typical 100mm range (the height of the milk container), there is a factor of around 10 between the range convergence time for a 5% reflectance compared to a 88% reflectance.

This is because when the reflectance is low, the time of flight sensor needs to make more measurements before a time of flight measurement is generated, hence it takes longer to determine the distance. Thus, rather than directly measuring a light reflectance, a difference in measurement time can be used to indicate the reflectance of the bottom of the container and hence determine if there is milk present or not.

The time taken to obtain a time of flight measurement may in this way be used as a proxy for a reflectance measurement.

Instead of, or as well as, using the time taken to obtain a time of flight measurement, the change in reflectance may be derived from a rate of change of the time taken to make the of flight measurements.

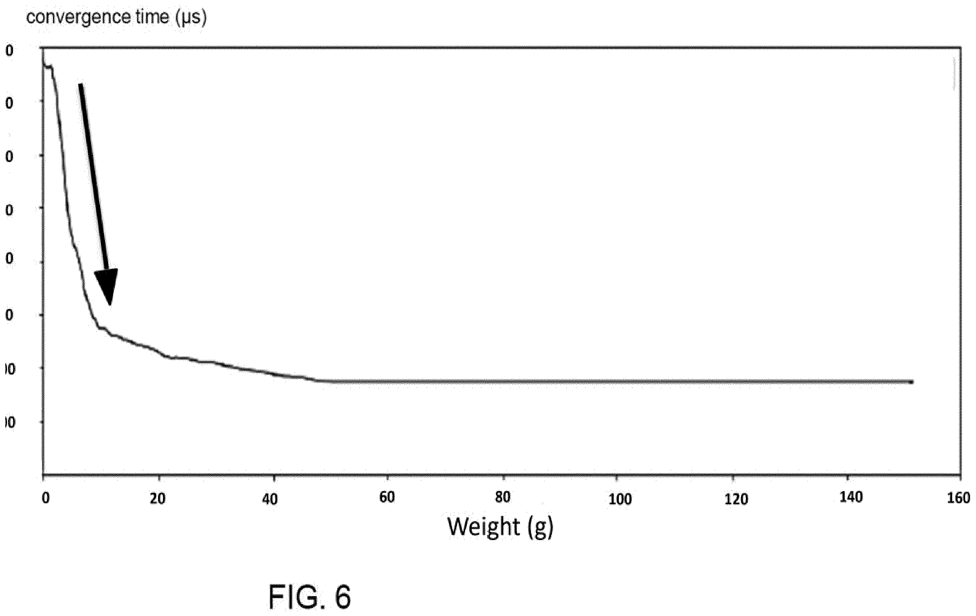

Figure 6 shows a graph of convergence time versus weight of collected milk.

As shown, the convergence time is initially approximately 0.75ms but it rapidly drops to around 0.3ms during the collection of the initial 10g of milk. Thus, rather than using absolute values of the convergence time, the steep negative initial slope may be used to detect the initial change in reflectance. This may provide tolerance when there is a small initial amount of residue in the milk vessel.

The change in convergence time is the result in the change of reflectance. Thus, whether reflectance values, convergence times or rate of change of convergence time is used as the measure, a change is detected from the first reflectance (of the empty container vessel base) to a milk reflectance, and thus identify when there is a change from an empty collection vessel to a collection vessel containing milk.

By way of example, the reflectance of milk in the IR wavelength range is for example in the range 50% to 70%. Thus a minimum reflectance of the milk in the selected wavelength of the sensor system may be 40%, for example 50% for example 60%.

The reflectance of the container to the IR wavelength can be made to be below 20%, for example below 15% for example below 10%, for example below 5% in the wavelength range of interest.

As mentioned above, by sensing the first expressed milk, the breast pump can be controlled between a stimulation setting and an expression setting.

The stimulation setting for example involves applying a first, relatively low level of vacuum (by which is meant a pressure only slightly below ambient pressure) at a first relatively high cycle rate (short cycles).

The expression setting then involves applying a second, relatively high level of vacuum (by which is meant a pressure below ambient pressure by a greater amount) at a second, relatively low cycle rate (long cycles).

Examples of typical pressure and cycle timings for the stimulation setting are -170mbar (-17kPa, i.e. 17kPa below atmospheric pressure) and a cycle of duration of 0.6 s. Typically, the pressure is in the range -10kPa to -20kPa with a cycle duration of less than 1.0s.

Examples of typical pressure and cycle timings for the expression settings are -250mbar (-25kPa, i.e. 25kPa below atmospheric pressure) and a cycle duration of 1.2s. Typically, the pressure is in the range --22kPa to -35kPa with a cycle duration of more than 1s, for example 1.0s to 1.5s.

These are just examples to give an indication of the typical difference between the expression mode and the stimulation mode.

The processing of the sensor signals may be performed at various possible locations. The processing may built in to the breast pump system, or integrated with the sensor system (for example it may be a clip-on feature) or in a remote device such as a mobile phone, or even hosted remotely in the cloud.

The example above makes use of a "dark" layer to absorb the radiation used by the sensor system.

In the case of visible or infrared light, the dark layer may be a black absorbing layer. If IR radiation is used for the sensing, the "dark" layer may be transparent to at least some visible light, and some transparent plastics are IR absorbing.

However, other parts of the electromagnetic spectrum may be used. The vessel is in particular preferably designed such that for the radiation being used, the reflectance of the at least the base of the vessel when empty is significantly different to the reflectance of milk.

The absorbing layer may be a coating on the outside of the milk container, or the material of the entire milk container may have the desired absorption characteristics. It will need to meet food standards, particularly if the absorbing layer is in contact with the milk in the container. The absorbing material may instead be part of a support on which the milk container is seated, in use. In such a case, the milk container itself may be transparent to the radiation used.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A collection vessel system (40) for use with a breast pump, comprising:
a collection vessel (42) for collecting milk expressed during use of the breast pump, the collection vessel having a base (44); and
a sensor system (50) for sensing the start of milk expression into the collection vessel,
wherein the sensor system comprises an emitter (52) for emitting electromagnetic radiation towards the base (44) and a sensor (54) for receiving electromagnetic radiation reflected from the base,
**characterized in that**:
the base (44) of the collection vessel when empty has a first reflectance to the emitted electromagnetic radiation below 20%; or
the collection vessel system comprises a support on which the collection vessel is mounted in use, and the combination of the base (44) of the collection vessel and the support, when the collection vessel is empty, has a first reflectance to the emitted electromagnetic radiation below 20%, and
wherein the sensor system is adapted to detect a change from the first reflectance to a milk reflectance, thereby to identify when there is a change from an empty collection vessel to a collection vessel containing milk.

2. The collection vessel system of claim 1, wherein the emitter (52) is for emitting visible light and the sensor (54) is for detecting visible light.

3. The collection vessel system of claim 1, wherein the emitter (52) is for emitting infrared light and the sensor (54) is for detecting infrared light.

4. The collection vessel system of claim 1, wherein the sensor system comprises a time of flight measurement system.

5. The collection vessel system of claim 4, wherein the sensor system is adapted to record a time taken thereby to make a time of flight measurement and to derive the change in reflectance from the time taken to make the time of flight measurement.

6. The collection vessel system of claim 5, wherein the sensor system is adapted to derive the change in reflectance from a rate of change of the time taken to make the of flight measurements.

7. The collection vessel system of any one of claims 1 to 6, wherein the collection vessel system comprises the support on which the collection vessel is mounted in use, and the support comprises an absorbing layer (46).

8. A breast pump device, comprising
at least one breast receiving portion (5) configured to receive a breast of a user; and
the collection vessel system of any one of claims 1 to 7.

9. The breast pump device of claim 8, comprising:
a pressure source (16) coupled to the at least one breast receiving portion; and
a controller (10) configured to control an operation of the pressure source in a stimulation mode and an expression mode,
wherein the controller is configured to switch from a stimulation mode to an expression mode in response to a signal from the sensor system of the collection vessel system.

10. The breast pump device of claim 8 or 9, comprising a wearable breast pump.

11. A computer program comprising computer program code which is adapted, when said program is run on the controller of the breast pump device of claim 9 to implement a method comprising:
determining the start of milk expression into a collection vessel system when using the breast pump device based on received data from a sensor system which senses a change in reflectance of a base of a collection vessel of the collection vessel system to electromagnetic radiation used by the sensor system from below 20% to a reflectance indicative of a collection vessel containing milk; and
generating an output based on the determined start of milk expression.

12. The computer program of claim 11, wherein the method comprises using the generated output to control the breast pump device to switch from a stimulation mode to an expression mode in response to the determined start of milk expression.

13. A controller for determining a start of milk collection into a collection vessel, wherein the controller is adapted to:
receive data from a sensor system, sensing a change in reflectance of a base of the collection vessel to electromagnetic radiation used by the sensor system from below 20% to a reflectance indicative of a collection vessel containing milk; and
interpret the received data to determine the start of milk collection and generate an output based on the determined start of milk collection.

14. The controller of claim 13, adapted to interpret the received data by analyzing convergence times of time of flight sensor data.

## Patentansprüche

1. Sammelbehältersystem (40) zur Verwendung mit einer Brustpumpe, umfassend:
einen Sammelbehälter (42) zum Sammeln der während der Verwendung der Brustpumpe ausgedrückten Milch, wobei der Sammelbehälter eine Basis (44) aufweist; und
ein Sensorsystem (50) zum Erfassen des Starts der Milchausdrückung in den Sammelbehälter,
wobei das Sensorsystem einen Emitter (52) zum Emittieren von elektromagnetischer Strahlung in Richtung der Basis (44) und einen Sensor (54) zum Empfangen der von der Basis reflektierten elektromagnetischen Strahlung umfasst,
**dadurch gekennzeichnet, dass**:
die Basis (44) des Sammelbehälters, wenn sie leer ist, einen ersten Reflexionsgrad für die emittierte elektromagnetische Strahlung von unter 20% aufweist; oder
das Sammelbehältersystem einen Träger umfasst, auf dem der Sammelbehälter im Gebrauch montiert ist, und die Kombination aus der Basis (44) des Sammelbehälters und dem Träger, wenn der Sammelbehälter leer ist, einen ersten Reflexionsgrad für die emittierte elektromagnetische Strahlung unter 20% aufweist, und
wobei das Sensorsystem adaptiert ist, um eine Änderung des ersten Reflexionsgrades in einen Milchreflexionsgrad zu detektieren und dadurch zu identifizieren, wenn ein Wechsel von einem leeren Sammelbehälter zu einem Milch enthaltenden Sammelbehälter stattfindet.

2. Sammelbehältersystem nach Anspruch 1, wobei der Emitter (52) zum Emittieren von sichtbarem Licht und der Sensor (54) zum Detektieren von sichtbarem Licht dient.

3. Sammelbehältersystem nach Anspruch 1, wobei der Emitter (52) dazu dient, Infrarotlicht zu emittieren und der Sensor (54) dazu dient, Infrarotlicht zu detektieren.

4. Sammelbehältersystem nach Anspruch 1, wobei das Sensorsystem ein Flugzeitmessungssystem umfasst.

5. Sammelbehältersystem nach Anspruch 4, wobei das Sensorsystem adaptiert ist, um eine Zeit aufzuzeichnen, die dadurch benötigt wird, um eine Flugzeitmessung durchzuführen, und um die Änderung des Reflexionsgrades aus der Zeit abzuleiten, die benötigt wird, um die Flugzeitmessung durchzuführen.

6. Sammelbehältersystem nach Anspruch 5, wobei das Sensorsystem adaptiert ist, um die Änderung des Reflexionsgrads aus einer Änderungsrate der für die Flugzeitmessung benötigten Zeit ableitet.

7. Sammelbehältersystem nach einem der Ansprüche 1 bis 6, wobei das Sammelbehältersystem den Träger umfasst, auf dem der Sammelbehälter im Gebrauch montiert ist, und der Träger eine absorbierende Schicht (46) umfasst.

8. Brustpumpenvorrichtung, umfassend
mindestens einen Brustempfangsabschnitt (5), der konfiguriert ist, um eine Brust eines Benutzers zu empfangen; und
das Sammelbehältersystem nach einem der Ansprüche 1 bis 7.

9. Brustpumpenvorrichtung nach Anspruch 8, umfassend:
eine Druckquelle (16), die mit dem mindestens einen Brustempfangsabschnitt gekoppelt ist; und
eine Steuereinheit (10), die konfiguriert ist, um einen Betrieb der Druckquelle in einem Stimulationsmodus und einem Ausdrückungsmodus zu steuern,
wobei die Steuereinheit konfiguriert ist, um als Antwort auf ein Signal von dem Sensorsystem des Sammelbehältersystems von einem Stimulationsmodus in einen Ausdrückungsmodus zu wechseln.

10. Brustpumpenvorrichtung nach Anspruch 8 oder 9, umfassend eine tragbare Brustpumpe.

11. Computerprogramm, umfassend einen Computerprogrammcode, der adaptiert ist, wenn das Programm auf der Steuereinheit der Brustpumpenvorrichtung nach Anspruch 9 ausgeführt wird, um ein Verfahren zu implementieren, das folgendes umfasst:
Bestimmen des Starts der Milchausdrückung in ein Sammelbehältersystem, wenn die Brustpumpvorrichtung verwendet wird, basierend auf empfangenen Daten von einem Sensorsystem, das eine Änderung des Reflexionsgrads einer Basis eines Sammelbehälters des Sammelbehältersystems gegenüber elektromagnetischer Strahlung, die von dem Sensorsystem verwendet wird, von unter 20% auf einen Reflexionsgrad, der angibt, dass ein Sammelbehälter Milch enthält, erfasst; und
Erzeugen einer Ausgabe basierend auf dem bestimmten Start der Milchausdrückung.

12. Computerprogramm nach Anspruch 11, wobei das Verfahren die Verwendung der erzeugten Ausgabe zum Steuern der Brustpumpenvorrichtung zum Umschalten von einem Stimulationsmodus in einen Ausdrückungsmodus als Antwort auf den bestimmten Start der Milchexpression umfasst.

13. Steuereinheit zum Bestimmen des Starts des Milchsammelns in einen Sammelbehälter, wobei die Steuereinheit adaptiert ist, um:
Daten von einem Sensorsystem zu empfangen, das eine Änderung des Reflexionsgrades einer Basis des Sammelbehälters gegenüber der von dem Sensorsystem verwendeten elektromagnetischen Strahlung von unter 20% auf einen Reflexionsgrad erfasst, der angibt, dass der Sammelbehälter Milch enthält; und
die empfangenen Daten zu interpretieren, um den Start des Milchsammelns zu bestimmen und eine Ausgabe basierend auf dem bestimmten Start des Milchsammelns erzeugen.

14. Steuereinheit nach Anspruch 13, adaptiert, um die empfangenen Daten durch Analysieren der Konvergenzzeiten der Flugzeitsensordaten zu interpretieren.

## Revendications

1. Système de récipient de collecte (40) destiné à être utilisé avec un tire-lait, comprenant :
un récipient de collecte (42) pour collecter du lait exprimé pendant l'utilisation du tire-lait, le récipient de collecte présentant une base (44) ; et
un système de capteur (50) pour détecter le début de l'expression de lait dans le récipient de collecte,
dans lequel le système de capteur comprend un émetteur (52) pour émettre un rayonnement électromagnétique vers la base (44) et un capteur (54) pour recevoir un rayonnement électromagnétique réfléchi par la base,
**caractérisé en ce que** :
la base (44) du récipient de collecte lorsqu'il est vide présente une première réflectance au rayonnement électromagnétique émis inférieure à 20 % ; ou
le système de récipient de collecte comprend un support sur lequel le récipient de collecte est monté en cours d'utilisation, et la combinaison de la base (44) du récipient de collecte et du support, lorsque le récipient de collecte est vide, présente une première réflectance au rayonnement électromagnétique émis inférieure à 20 %, et
dans lequel le système de capteur est adapté pour détecter un changement de la première réflectance à une réflectance de lait, pour identifier ainsi lorsqu'il y a un changement d'un récipient de collecte vide à un récipient de collecte contenant du lait.

2. Système de récipient de collecte selon la revendication 1, dans lequel l'émetteur (52) est destiné à émettre de la lumière visible et le capteur (54) est destiné à détecter de la lumière visible.

3. Système de récipient de collecte selon la revendication 1, dans lequel l'émetteur (52) est destiné à émettre de la lumière infrarouge et le capteur (54) est destiné à détecter de la lumière infrarouge.

4. Système de récipient de collecte selon la revendication 1, dans lequel le système de capteur comprend un système de mesure de temps de vol.

5. Système de récipient de collecte selon la revendication 4, dans lequel le système de capteur est adapté pour enregistrer un temps pris ainsi pour effectuer une mesure de temps de vol et pour dériver le changement de réflectance à partir du temps pris pour effectuer la mesure de temps de vol.

6. Système de récipient de collecte selon la revendication 5, dans lequel le système de capteur est adapté pour dériver le changement de réflectance à partir d'un taux de changement du temps pris pour effectuer les mesures de vol.

7. Système de récipient de collecte selon l'une quelconque des revendications 1 à 6, dans lequel le système de récipient de collecte comprend le support sur lequel le récipient de collecte est monté en cours d'utilisation, et le support comprend une couche absorbante (46).

8. Dispositif de tire-lait comprenant
au moins une partie de réception de sein (5) configurée pour recevoir un sein d'une utilisatrice ; et
le système de récipient de collecte selon l'une quelconque des revendications 1 à 7.

9. Dispositif de tire-lait selon la revendication 8, comprenant :
une source de pression (16) couplée à au moins une partie de réception de sein ; et
un dispositif de commande (10) configuré pour commander un fonctionnement de la source de pression dans un mode de stimulation et un mode d'expression,
dans lequel le dispositif de commande est configuré pour basculer d'un mode de stimulation à un mode d'expression en réponse à un signal provenant du système de capteur du système de récipient de collecte.

10. Dispositif de tire-lait selon la revendication 8 ou 9, comprenant un tire-lait portable.

11. Programme informatique comprenant du code de programme informatique qui est adapté, lorsque ledit programme est exécuté sur un dispositif de commande du dispositif de tire-lait selon la revendication 9, pour mettre en œuvre un procédé comprenant :
la détermination du début de l'expression de lait dans un système de récipient de collecte lors de l'utilisation du dispositif de tire-lait sur la base des données reçues provenant d'un système de capteur qui détecte un changement de réflectance d'une base d'un récipient de collecte du système de récipient de collecte au rayonnement électromagnétique utilisé par le système de capteur inférieur à 20 % à une réflectance indiquant un récipient de collecte contenant du lait ; et
la génération d'une sortie sur la base du début déterminé de l'expression de lait.

12. Programme informatique selon la revendication 11, dans lequel le procédé comprend l'utilisation de la sortie générée pour commander le dispositif de tire-lait pour basculer d'un mode de stimulation à un mode d'expression en réponse au début déterminé de l'expression de lait.

13. Dispositif de commande destiné à déterminer le début d'une collecte de lait dans un récipient de collecte, dans lequel le dispositif de commande est adapté pour :
recevoir des données provenant d'un système de capteur, détectant un changement de réflectance d'une base du récipient de collecte au rayonnement électromagnétique utilisé par le système de capteur inférieur à 20 % à une réflectance indiquant un récipient de collecte contenant du lait ; et
interpréter les données reçues pour déterminer le début d'une collecte de lait et générer une sortie sur la base du début déterminé de la collecte de lait.

14. Dispositif de commande selon la revendication 13, adapté pour interpréter les données reçues en analysant des temps de convergence de données de capteur de temps de vol.
